# EUROPÄISCHE PATENTSCHRIFT

(11) **EP 1 061 064 B1**
(45) Veröffentlichungstag und Bekanntmachung des Hinweises auf die Patenterteilung: **25.06.2003**
(21) Anmeldenummer: 00112508.7
(22) Anmeldetag: 13.06.2000
(51) Int. Cl.: C07C 51/367, C07C 59/305, C07C 59/60, C07C 41/03

(54) **Verfahren zur Herstellung von Ethercarbonsäuren mit niedrigem Restalkoholgehalt**
Process for the production of ethercarboxylic acids with low rest alcohol content
Procédé de préparation d' acides ether carboxyliques à faible teneur en alcool

(30) Priorität: 19.06.1999 DE 19928128
(43) Veröffentlichungstag der Anmeldung: 20.12.2000
(73) Patentinhaber: Clariant GmbH, 65929 Frankfurt am Main (DE)
(72) Erfinder: Klug, Peter, Dr., 63762 Grossostheim (DE); Kupfer, Rainer, Dr., 65795 Hattersheim (DE); Wimmer, Ignaz, 84543 Winhöring (DE); Winter, Rüdiger, 65929 Frankfurt (DE)

(56) Entgegenhaltungen:
- EP-A- 0 166 958
- US-A- 4 223 163
- US-A- 4 239 917
- US-A- 5 233 087

## Beschreibung

Die vorliegende Erfindung betrifft ein neuartiges Verfahren zur Herstellung von Ethercarbonsäuren mit niedrigem Restalkoholgehalt.

Ethercarbonsäuren, d.h. organische Carbonsäuren, die neben der Carboxylfunktion eine oder mehrere Etherbrücken tragen, bzw. deren Alkali- oder Aminsalze; sind als milde Detergenzien mit hohem Kalkseifendispergiervermögen bekannt. Sie finden sowohl in Waschmittel- und Kosmetikformulierungen, aber auch in technischen Anwendungen (z.B. Metallbearbeitungsflüssigkeiten, Kühlschmiermittel) Verwendung. Diese Produkte werden gemäß dem Stand der Technik entweder durch Alkylierung von Alkohol- oder Fettalkoholoxethylaten oder -oxpropylaten mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren hergestellt. Der Nachteil der Williamson-Synthese besteht in dem nicht vollständigem Umsatz des zugrundeliegenden Oxethylats zur Ethercarbonsäure bzw. deren Salz. Die Umsätze liegen trotz Überschüssen an Chloressigsäurederivat oft nur zwischen 70-85 %. Es verbleiben im Endprodukt Restmengen an Oxethylat bzw. an dem Fettalkohol, der dem Oxethylat zugrundeliegt, da dieser mit Chloressigsäure bzw. Chloressigsäure-Na-salz langsamer reagiert als ein oxethylierter Alkohol. Besonders ungünstig ist dies, wenn man niedrigoxethylierte Alkohole als Basismaterial benutzt. Diese Oxethylate können zwischen 5 und 30 % Restfettalkohol enthalten. Da der Fettalkohol gegenüber einem oxethylierten Fettalkohol eine geringere Reaktivität in der Williamson-Synthese aufweist führt dies ebenfalls zu einem hohen Restfettalkoholgehalt in der Ethercarbonsäure und gleichzeitig zu schlechteren Umsätzen zur Ethercarbonsäure. Verwendet man hierbei Oxethylate niedrigerer Fettalkohole mit Kettenlängen von C₅-C₁₂, so treten aus diesem Grund oft Geruchsprobleme durch die Restalkoholgehalte auf, da diese Fettalkohole geruchsintensiv sind.

Deshalb besteht Bedarf an Verfahren, die den Restalkoholgehalt in der Ethercarbonsäure reduzieren. Dies kann z.B. durch Verwendung von klassischen narrow-range-Katalysatoren bei der Oxethylierung geschehen, die den Restgehalt an Fettalkohol im Oxethylat und damit auch den Fettalkoholgehalt in der Ethercarbonsäure erniedrigen. Allerdings werden als Katalysator, wie z.B. in der EP-A-0 295 578 offenbart, oft Salze von verschiedenen Carbonsäuren mit mehrwertigen Ionen (z.B. Calcium) verwendet, die wieder abgetrennt werden müssen oder Probleme durch Trübungen verursachen. Zusätzlich muß dieser Katalysator in einer vorgeschalteten Reaktionsstufe hergestellt werden.

US-4 223 163 offenbart ein Verfahren zur Herstellung von ethoxilierten Ethercarbonsäuren, indem man Alkalimetalle oder Alkalimetallhydride als Katalysatoren für die Ethoxilierung der Fettalkohole verwendet. Bei diesem Verfahren entstehen stöchiometrische Mengen an Wasserstoff.

Überraschenderweise wurde nun gefunden, daß diese Problematik auf einfachem Wege zu umgehen ist, wenn man Fettalkohole zunächst unter Einsatz nichtkatalytischer Mengen an Alkalikatalysator (NaOH, KOH, Alkoholate über 5 mol-%) mit Alkylenoxiden umsetzt und die resultierenden hochalkalischen Reaktionsmischungen, die aus einem Gemisch von oxethylierten Alkoholen und Alkoholaten verschiedener Polyalkylenglykolether bestehen, anschließend in einer klassischen Williamson-Synthese mit Natriumchloracetat in die entsprechende Ethercarbonsäure überführt. Hierdurch wird der Restgehalt an Fettalkohol in der Ethercarbonsäure ohne spezielle Katalysatoren deutlich verringert, was u.a. auch zu geruchlich verbesserten Produkten führt. Zusätzlich erhält man eine narrow-range-Verteilung der resultierenden Ethercarbonsäure, was zu vorteilhaften Eigenschaftsprofilen (z.B. besserer Löslichkeit) führen kann.

Gegenstand der Erfindung ist ein Verfahren zur Herstellung von Ethercarbonsäuren oder deren Alkali- oder Ammoniumsalzen mit niedrigem Restalkoholgehalt, dadurch gekennzeichet, daß man einen ein- oder mehrwertigen Alkohol zunächst mit einer unterstöchiometrischen, zwischen 5 und 95 mol-% liegenden Menge einer basischen Verbindung, ausgewählt aus Erdalkalimetallhydroxiden, Alkalimetallhydroxiden und Alkalimetallalkoholaten, in das entsprechende Alkoholat überführt, anschließend mit Alkylenoxiden umsetzt und die hochalkalische, mehr als 5 mol-% oxalkylierte Alkoholate enthaltende Reaktionsmischung, ggf. nach Abdestillieren des verbleibenden Restalkohols, direkt mit einem Chloressigsäurederivat alkyliert und ggf. durch Ansäuern mit Mineralsäure in die freie Ethercarbonsäure überführt.

Als Basisfettalkohol für das hier beschriebene Verfahren eignen sich lineare oder verzweigte, gesättigte oder ungesättigte Alkohole mit 1 bis 30 C-Atomen, beispielsweise Fettalkohole mit 1-30 C-Atomen sowie Alkylphenole mit einem C₁-C₂₀-Alkylrest. Bevorzugt sind C₆-C₂₂-Fettalkohole.

Obige Alkohole werden im erfindungsgemäßen Verfahren mit Alkylenoxiden, z.B. Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf Fettalkohol werden 1-30 mol Alkylenoxid beaufschlagt, bevorzugt 1-12 mol.

Als basische Verbindung zur Herstellung der oxethylierten Alkoholate werden Erdalkali-/Alkalimetallhydroxide oder-alkoholate (Natriummethylat, Natriumethylat, Kalium-tert.-butylat) verwendet , bevorzugt sind aber Alkalimetallhydroxide, besonders Natriumhydroxid oder Kaliumhydroxid.

Die basischen Verbindungen werden in Mengen von ca. 5-95 mol-% bezogen auf den ein- oder mehrwertigen Alkohol eingesetzt, bevorzugt zwischen 15 und 90 mol-%, besonders bevorzugt zwischen 20-60 mol-%.

Ausgehend vom Basisalkohol werden die zur Oxalkylierung notwendigen Alkoholate durch Umsetzung mit den basischen Verbindungen hergestellt. Um höhere Anteile an Nebenprodukten (Glykole, Glykolether niederer Alkohole) im Endprodukt zu vermeiden, sollte das hierbei entstehende Reaktionswasser oder der entsprechende niedere Alkohol aus dem Reaktionsgemisch vor der Umsetzung mit dem Alkylenoxid entfernt werden. Dies kann entweder durch Umsetzung des Alkohols mit einem Alkalihydroxid und Abdestillieren des Reaktionswassers geschehen oder durch Umsetzung des Basisalkohols mit einem Alkoholat eines niederen Alkohols und Abdestillieren des niederen Alkohols. Im ersteren Fall kann der Fettalkohol auch als Wasserschlepper für das Reaktionswasser dienen.

Die erhaltene Mischung aus Fettalkohol und dem entsprechenden Alkoholat wird dann mit ca. 1-30 mol eines Alkylenoxids, bevorzugt Ethylenoxid und/oder Propylenoxid umgesetzt, die Reaktionstemperaturen liegen hierbei bei ca. 80-160 °C. Dabei tritt im Vergleich zu einer mit niedrigen Alkalimengen katalysierten Reaktion eine engere Homologenverteilung ein, die zu einer Verminderung der Restalkoholmenge führt. Gegebenenfalls kann man den noch verbleibenden Restalkohol zusätzlich noch unter vermindertem Druck abdestillieren.

Im anschließenden Reaktionsschritt wird die Alkoholat/Alkoholoxethylat-Mischung mit einem Chloressigsäurederivat und einer Base, bevorzugt trockenem Chloressigsäure-Natriumsalz und Natriumhydroxid umgesetzt. Dies kann geschehen, indem man die Oxethylat/Alkoholat-Mischung mit 100-150 mol-% Natriumchloracetat bei 30-100 °C umsetzt und gleichzeitig oder nacheinander mit festem Natriumhydroxid oder Kaliumhydroxid versetzt, so daß die Summe aus der in der Oxethylat-/Alkoholatmischung bereits vorliegenden Base und der zusätzlich zugegebenen Basenmenge der Menge an Natriumchloracetat entspricht. Die aus der Umsetzung mit dem Alkylenoxid bereits enthaltene Basenmenge kann somit direkt für die anschließende Williamson-Synthese genutzt werden und muß nicht, wie bei der Synthese eines Standard-Oxethylats, ausgewaschen werden.

Anschließend an die Alkylierungsreaktion kann die entstehende Lösung des Ethercarbonsäure-Alkalisalzes entweder direkt als Detergenz Verwendung finden oder zur Reduzierung des Salzgehaltes in die freie Ethercarbonsäure überführt werden. Hierzu wird mit starker Mineralsäure (Salzsäure, Schwefelsäure) auf pH < 3 angesäuert und die Ethercarbonsäure durch Phasentrennung oberhalb ihres Trübungspunktes heiß als Oberphase abgetrennt.

Wie die folgenden Versuchsbeispiele zeigen, können nach dem hier gezeigten Verfahren Ethercarbonsäuren oder Ethercarbonsäuresalze mit engerer Homologenverteilung und höheren Umsätzen mit geringeren Restalkoholgehalten erhalten werden.

### Beispiel 1:

### Isononylalkolhol + 3 EO-Ethercarbonsäure

In einer 4 l-Rührapparatur mit Wasserabscheider werden 2163 g (15,0 mol) Isononylalkohol und 150 g (3,75 mol) Natriumhydroxid unter Stickstoff vorgelegt und die Reaktionsmischung auf 150 °C aufgeheizt. Anschließend wird bis zum Sieden langsam Vakuum angelegt und so reguliert, daß die Siedetemperatur immer 150°C beträgt, wobei insgesamt 62,3 g (3,46 mol) Wasser ausgekreist werden.
Siedebeginn ist bei ca. 600 mbar. Das Endvakuum beträgt ca. 180 mbar, wird nach ca. 1 h erreicht und dann für ca. 2 h gehalten.

In einer 5 I-Ethoxylierungsapparatur werden 2252 g (15,0 mol) des obigen Isononylalkohol/ Isononylalkoholat-Gemisches unter 1 bar Stickstoff vorgelegt und auf 160°C aufgeheizt. Dann werden 1982 g (45,0 mol) Ethylenoxid in ca. 1,5 h bei 160°C zudosiert, Nachreaktion 1 h bei 160°C bis zur Druckkonstanz. Der restliche, enthaltene Isononylalkohol wird bei 125 °C Sumpftemperatur und 25 mbar Vakuum abdestilliert (gesamt 462 g).

In einer 10 l-Rührapparatur werden 1886 g (5,9 mol) des obigen Isononylalkohol/Isononylalkoholat-Ethoxylat-Destillationsrückstands unter Stickstoff vorgelegt und auf 40°C aufgeheizt. Dann werden 825 g (7,10 mol) Natriumchloracetat eingetragen und anschließend die Reaktionsmischung auf 50°C aufgeheizt. Nach jeweils 45 min werden 208 g (5,20 mol) NaOH-Microprills in drei gleichgroßen Portionen bei 50°C eingetragen. Nachreaktion 45 min bei 50°C und 2 h bei 70°C. Danach werden 3259 g (7,15 mol) Salzsäure 8 % zulaufen lassen, die Mischung wird auf 95°C aufgeheizt und in eine 10 l-Rührapparatur mit Bodenablaß überführt. Die Phasentrennung erfolgt nach 15 min bei 95°C, wobei ca. 3816 g wäßrige Unterphase abgetrennt werden. Es werden 2391 g Isononylalkohol + 3 EO-Ethercarbonsäure erhalten.

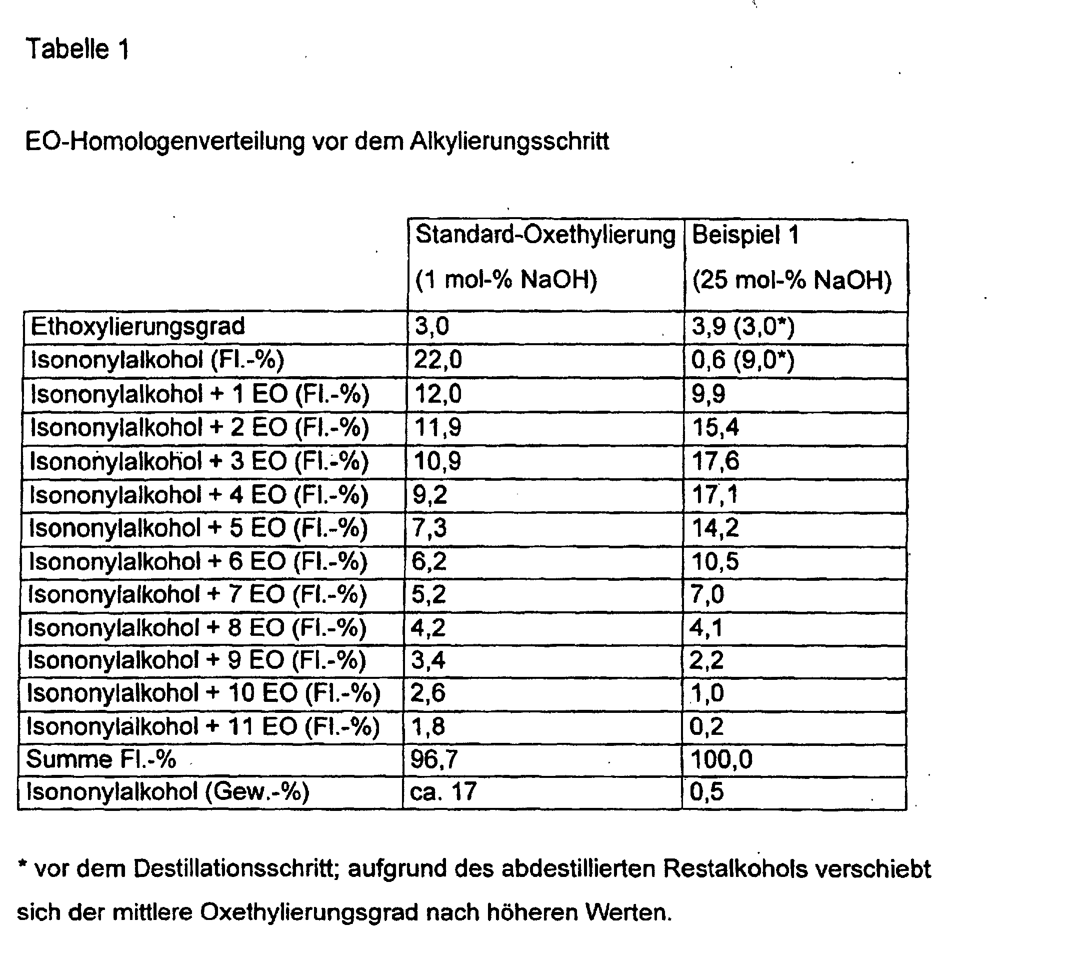

### Kennzahlen der Ethercarbonsäure

| | Säurezahl (mg KOH / g) | Gehalt nach Säurezahl (%) | Katalysator / Menge |
|---|---|---|---|
| Ethercarbonsäure auf Basis Standardoxethylat | 126,0 (3,0 EO) | 76,4 | NaOMe 1,0 Mol-% |
| Beispiel 1 | 130,6 (3,9 EO) | 87,1 | NaOH 25 Mol-% |

### Beispiel 2:

### Ethercarbonsäure auf Basis Oleylalkohol + 5 EO

Es wurde Oleylalkohol analog zu Beispiel 1 unter Verwendung von 50 mol-% NaOH als Katalysator umgesetzt, der verbleibende Restalkoholgehalt wurde aber im Gegensatz zu Beispiel 1 nicht abdestilliert.

Die folgende Tabelle zeigt die EO-Homologenverteilung vor dem abschließenden Alkylierungsschritt:

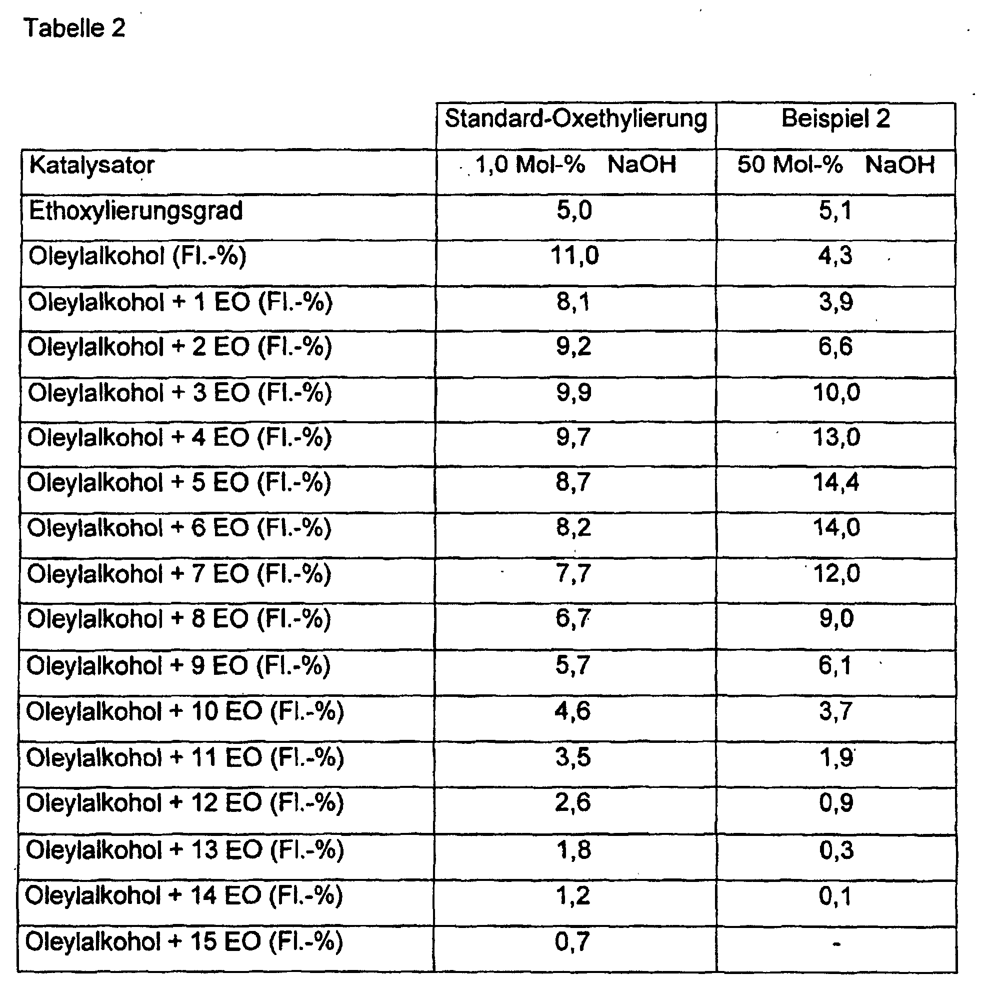

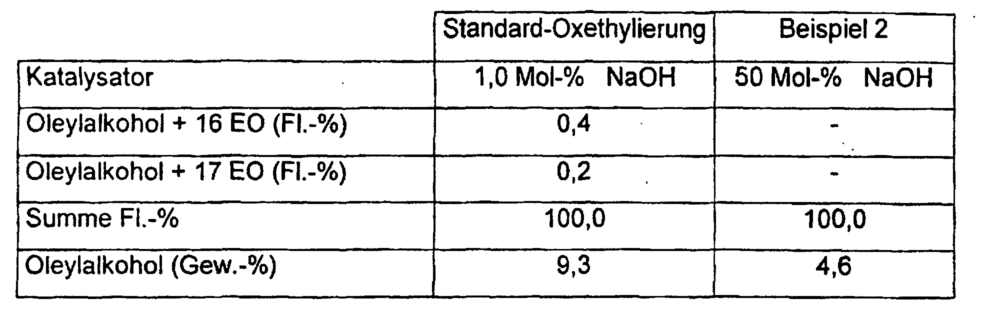

### Kennzahlen der Ethercarbonsäure:

| | Säurezahl (mg KOH / g) | Gehalt nach Säurezahl (%) |
|---|---|---|
| Beispiel 2 | 91,3 | 88,7 |
| Ethercarbonsäure auf Basis Standardoxethylat | 83,4 | 79,3 |

## Patentansprüche

1. Verfahren zur Herstellung von Ethercarbonsäuren oder deren Alkali- oder Ammoniumsalzen mit niedrigem Restalkoholgehalt, dadurch gekennzeichet, daß man einen ein- oder mehrwertigen Alkohol zunächst mit einer unterstöchiometrischen, zwischen 5 und 95 mol-% liegenden Menge einer basischen Verbindung, ausgewählt aus Erdalkalimetallhydroxiden, Alkalimetallhydroxiden und Alkalimetallalkoholaten, in das entsprechende Alkoholat überführt, anschließend mit Alkylenoxiden umsetzt und die hochalkalische, mehr als 5 mol-% oxalkylierte Alkoholate enthaltende Reaktionsmischung, ggf. nach Abdestillieren des verbleibenden Restalkohols, direkt mit einem Chloressigsäurederivat alkyliert und ggf. durch Ansäuern mit Mineralsäure in die freie Ethercarbonsäure überführt.

2. Verfahren nach Anspruch 1, wobei der ein- oder mehrwertige Alkohol insgesamt 1 bis 30 Kohlenstoffatome umfaßt.

3. Verfahren nach Anspruch 2, wobei es sich um einen C₆-C₂₂-Fettalkohol handelt.

4. Verfahren nach einem oder mehreren der Ansprüche 1 bis 3, wobei das Alkylenoxid Ethylen-, Propylen- oder Butylenoxid ist.

5. Verfahren nach einem oder mehreren der Ansprüche 1 bis 4, wobei die Menge der basischen Verbindung zwischen 15 und 90 mol-%, bezogen auf die Menge des ein- oder mehrwertigen Alkohols, liegt.

## Claims

1. A process for the preparation of ether carboxylic acids or alkali metal or ammonium salts thereof with low residual alcohol content, which comprises firstly converting a mono- or polyhydric alcohol into the corresponding alkoxide using a substoichiometric amount between 5 and 95 mol% of a basic compound chosen from the group consisting of alkaline earth metal hydroxides, alkali metal hydroxides and alkali metal alkoxides, and then reacting the alkoxide with alkylene oxides, and, if necessary after distilling off the residual alcohol which remains, alkylating the highly alkaline reaction mixture, which comprises more than 5 mol% of alkoxylated alkoxides, directly with a chloroacetic acid derivative, and, if necessary, converting the alkylated product into the free ether carboxylic acid by acidification with mineral acid.

2. The process as claimed in claim 1, where the mono-or polyhydric alcohol contains a total of from 1 to 30 carbon atoms.

3. The process as claimed in claim 2, where the alcohol is a C₆-C₂₂-fatty alcohol.

4. The process as claimed in one or more of claims 1 to 3, where the alkylene oxide is ethylene oxide, propylene oxide or butylene oxide.

5. The process as claimed in one or more of claims 1 to 4, where the amount of basic compound is between 15 and 90 mol%, based on the amount of mono- or polyhydric alcohol.

## Revendications

1. Procédé de préparation d'acides éthercarboxyliques ou de leurs sels de métaux alcalins ou d'ammonium, ayant une faible teneur en alcool résiduel, **caractérisé en ce qu'**on convertit en l'alcoolate correspondant un mono- ou polyalcool, d'abord avec une quantité inférieure à la quantité stoechiométrique, comprise entre 5 et 95 % en moles, d'un composé basique choisi parmi les hydroxydes de métaux alcalino-terreux, les hydroxydes de métaux alcalins et les alcoolates de métaux alcalins, puis on le fait réagir avec des oxydes d'alkylène, et on alkyle directement avec un dérivé de l'acide chloracétique, éventuellement après avoir chassé par distillation l'alcool résiduel restant, le mélange réactionnel, qui est très alcalin et qui contient plus de 5 % en moles d'alcoolates alcoxylés, et, éventuellement, par acidification avec un acide minéral, on le convertit en l'acide éthercarboxylique libre.

2. Procédé selon la revendication 1, dans lequel le mono- ou polyalcool contient en tout 1 à 30 atomes de carbone.

3. Procédé selon la revendication 2, dans lequel il s'agit d'un alcool gras en C₆-C₂₂.

4. Procédé selon l'une ou plusieurs des revendications 1 à 3, dans lequel l'oxyde d'alkylène est l'oxyde d'éthylène, de propylène ou de butylène.

5. Procédé selon l'une ou plusieurs des revendications 1 à 4, dans lequel la quantité du composé basique est de 15 à 90 % en moles par rapport à la quantité du mono- ou du polyalcool.
